# EUROPEAN PATENT APPLICATION

(11) **EP 3 299 038 A1**
(43) Date of publication of application: **28.03.2018**
(21) Application number: 17190439.4
(22) Date of filing: 11.09.2017
(51) Int. Cl.: A61L 27/18, A61L 31/06, A61L 31/10, A61L 31/12, A61L 31/14, A61L 31/16, A61K 9/00, A61K 47/10, A61K 9/20, C08L 67/04

(54) **PROLONGED DRUG-ELUTING PRODUCTS**

(30) Priority: 23.09.2016 US 201615274105
(71) Applicant: Micell Technologies, Inc., Durham, NC 27713 (US)
(72) Inventor: KIORPES, Timothy Charles, DOYLESTOWN, PA Pennsylvania 18902 (US); MCCLAIN, James B., Ocracoke, NC North Carolina 27960 (US)
(74) Representative: Regimbeau

(57) **Abstract**

A drug-eluting product is disclosed comprising an at least partially bioabsorbable element and an active pharmaceutical agent having a morphology, a solubility, and an average particle size which are selected so that the active pharmaceutical agent continues to dissolve during the biodegradation of the bioabsorbable element. The morphology is a crystalline, semi-crystalline or amorphous morphology, the solubility is less than 100 µg/ml and the average particle size is grater that about 100 nm.

## Description

### FIELD OF THE INVENTION

The present invention relates to drug-eluting products, including drug-eluting stents. More particularly, the present invention relates to drug-eluting products which include bioabsorbable components, such as implantable drug delivery depots for systemic or local drug delivery, and drug-eluting stents, which include bioabsorbable polymer components or coatings and/or bioabsorbable stent substrates themselves.

### BACKGROUND OF THE INVENTION

The treatment of various bodily disorders can oftentimes utilize local delivery of bioactive or active pharmaceutical agents or drugs in order to treat particular bodily disorders or interventional procedures. Such agents or drugs are often compounded with non-therapeutic chemicals; typically bioabsorbable polymers or readily metabolizable excipients. Such agents or drugs can be incorporated into a device used for such purposes in a variety of manners so it can be delivered directly to an afflicted region at or adjacent to a region of implantation.

Furthermore, in many of these treatment situations, the presence of non-therapeutic chemicals or a device is required only for a limited period of time. Therefore, such materials or devices can be composed, in whole or in part, of materials that degrade, absorb, erode, disintegrate, or metabolize through exposure to conditions within the body until the treatment regimen is completed. Examples of such devices include expandable endoprostheses which can be implanted in a bodily lumen, bioabsorbable orthopedic devices, bioabsorbable surgical aids (such as sutures), etc. In the case of expandable endoprosthesis, these correspond to artificial devices placed inside the body and the lumen can be a cavity or a tubular organ, such as a blood vessel.

One particular example of such devices includes stents, which generally function to hold open an expanded segment of a blood vessel or other anatomical lumen, such as urinary tracts and bile ducts. Stents are, for example, often used in treatment of atherosclerotic stenosis in blood vessels. Such stenosis refers to a narrowing or constriction of the diameter of a blood vessel or other orifice. In such treatments, stents generally hold open or reinforce these vessels and prevent restenosis following angioplasty in the vascular system. Restenosis relates to the recurrence of stenosis in a blood vessel or heart valve after it has been treated with apparent success.

It has thus been found useful to coat these biomedical implants to provide for the localized delivery of pharmaceutical or biological agents to target specific locations within the body for therapeutic or prophylactic benefit. Of particular interest, drug-eluting stents have become increasingly popular for these purposes. Typically, these pharmaceutical or biological agents are co-deposited with a polymer. The coating of these stents can thus provide for controlled release, including long-term or sustained release, of the pharmaceutical or biological agent providing a local therapeutic effect to the stented lumen. Examples of such coated stents include those disclosed in U.S. Patent Nos. 8,298,565 and 8,852,625 and U.S. Patent Publication Nos. 2010/0256748 and 2007/0008564 of the Assignee of the present application. These products can include, for example, polymers which are bioabsorbable, degradable, erodible, and/or resorbable (these terms are generally utilized interchangeably). The stents themselves are generally composed of scaffolding, including a pattern or network of interconnecting structural elements or struts, which can be formed from wires, tubes, or sheets of material in a cylindrical shape. Typically these stents are capable of being compressed or crimped onto a catheter so they can be delivered to or deployed at a treatment site. In many applications, the presence of the stent is only necessary for a limited period of time, after which the stent can pose a liability due to the presence of foreign material, poor healing, and/or precluding the return of the blood vessel to vasomotion, and therefore the development of bioresorbable stents of scaffolds have been realized. This eliminates the need for a permanent implant, such as a metal stent substrate or the like, in a vessel. Stents have thus been fabricated from biodegradable, bioabsorbable, and/or bioerodable materials, such as bioabsorbable polymers and bioabsorbable metals, which can be designed to completely erode after some period of time subsequent to the clinical need for them has ended. A discussion of the development of these stent scaffolds is set forth in "Bioresorbable Scaffold; the Advent of a New Era in Percutaneous Coronary and Peripheral Revascularization? "New Drugs and Technologies, Onuma, Circulation, February 22, 2011; 123:779-797, the disclosure of which is incorporated herein by reference thereto.

One of the problems with drug delivery compositions, and especially drug-eluting stents which include biodegradable components, such as biodegradable polymers and/or substrate structures themselves, is that the remnants, or more specifically the chemical species created over the course of degradation of the bioabsorbable material of the biodegradable portions tend to illicit a local inflammatory response in the associated bodily structure or blood vessels. Therefore, the search has continued for improved drug-eluting products which include biodegradable or bioabsorbable components.

### SUMMARY OF THE INVENTION

In accordance with the present invention, a drug-eluting product has been discovered which includes a degradable component having a predetermined period of degradation, and an active pharmaceutical agent having a particle size distribution which includes a preselected range of particle sizes selected so that the active pharmaceutical agent will be released over the entire predetermined period of degradation of the degradable component, and preferably substantially continuously over the entire period. More particularly, the pharmaceutical agent will be released for a period which extends beyond the period of degradation of the degradable component, and most preferably for a total period of at least about 3 months, preferably at least about 6 months, more preferably at least about 9 months, such as at least about 12 months, and most preferably at least about 18 months. The accomplishment of these results can employ a number of other factors which can be utilized in order to extend and selectively modify the rate and period of drug release. These include the morphology of the drug, primarily that it be in the crystalline or semi-crystalline state, and the poor solubility of the drug in the aqueous environment it will encounter in the body. Preferably, these drugs will have such a solubility of less than about 100 µg/ml, even more preferably less than about 75 µg/ml even more preferably less than about 50 µg/ml and most preferably less than about 10 µg/ml. Preferably, the degradable component will include a bioabsorbable polymer, a bioabsorbable metal, a degradable carrier, an excipient, and the like.

In accordance with one embodiment of the drug-eluting product of the present invention, the product itself is in the form of an implant, for either systemic or local delivery, such as a stent; or an oral pharmaceutical, including pills, capsules, tablets, and lozenges; injectables; trans-mucosals; inhalable products; and topical products, such as transdermals and the like.

In accordance with another embodiment of the drug-eluting product of the present invention, the drug-eluting product will preferably comprise a drug-eluting stent. Preferably, the degradable component will then include a bioabsorbable polymer, and more preferably one which will continue to degrade over an extended period of time, such as a period of more than about 10 days, and preferably more than about 6 months, and most preferably more than about one year.

In accordance with another embodiment of the drug-eluting product of the present invention, that active pharmaceutical agent has a particle size of greater than about 100 nm, and in a preferred embodiment, greater than about 0.5 microns.

In accordance with another embodiment of the drug-eluting product of the present invention, the active pharmaceutical agent includes a first portion of the active pharmaceutical agent having a particle size of between about 1 and 4 microns, and a second portion of the active pharmaceutical agent having a particle size of between about 2 and 10 microns.

In accordance with another embodiment of the drug-eluting product of the present invention, the degradable component comprises a substrate which includes a polymer coating, and preferably the polymer coating comprises a bioabsorbable polymer coating.

In accordance with another embodiment to the drug-eluting product of the present invention, the drug-eluting product includes a bioabsorbable polymer. Preferably, the bioabsorbable polymer is either disposed on or admixed with the degradable component.

In accordance with another embodiment of the present invention, a drug-eluting product has been discovered which includes an at least partially bioabsorbable element, and includes an active pharmaceutical agent which has an average particle size which is large enough so that the active pharmaceutical agent continues to dissolve during the biodegradation of the bioabsorbable element.

In accordance with a preferred embodiment of the present invention, the active pharmaceutical agent has an average particle size of greater than about 0.5 microns. In another embodiment, the active pharmaceutical agent has an average particle size between about 0.5 and 100 microns, particularly in the case of an oral pharmaceutical.

In accordance with this aspect of the present invention, the active pharmaceutical agent preferably includes a first portion of the active pharmaceutical agent having a particle size of between about 0.5 and 10 microns, and a second portion of the active pharmaceutical agent having a particle size of between about 1 and 100 microns, but preferably wherein the second portion of the active pharmaceutical agent being a greater particle size than that of the first portion of the pharmaceutical agent.

In accordance with one embodiment of the drug-eluting product of the present invention, the drug-eluting product comprises a drug-eluting stent.

In accordance with another embodiment of the drug-eluting product of the present invention, the drug-eluting product includes a degradable component, such as a substrate, and a polymer coating disposed on the degradable component. In a preferred embodiment, the at least partially bioabsorbable element comprises a bioabsorbable polymer coating disposed on the degradable component. In another embodiment, the at least partially bioabsorbable element comprises a bioabsorbable polymer coating disposed on the degradable component, and the degradable component comprises a bioabsorbable substrate. In a preferred embodiment, the bioabsorbable element comprises a resorbable polymer or metal. Further in accordance with this embodiment, the active pharmaceutical agent can be incorporated with the bioabsorbable polymer and/or the degradable component. For example, the same or two different active pharmaceutical agents can be incorporated in the bioabsorbable polymer and the degradable component. As an example, a shorter term dissolving drug can be incorporated in one of these components, such as the biodegradable polymer and a longer term dissolving drug incorporated in the other components, such as the degradable component.

In accordance with another embodiment of the drug-eluting product of the present invention, the degradable component is a bioabsorbable polymer, a bioabsorbable metal, a degradable carrier, an excipient, or a pill core.

In accordance with the present invention, a method for preparing a drug-eluting product has been discovered, which comprises providing an at least partially bioabsorbable element, and including an active pharmaceutical agent in the at least partially bioabsorbable element, the active pharmaceutical agent having an average particle size large enough so that the active pharmaceutical agent continues to dissolve during the biodegradation of the bioabsorbable element.

In accordance with a preferred embodiment of the method of the present invention, the active pharmaceutical agent has an average particle size greater than about 2 microns. In another embodiment, the active pharmaceutical agent has an average particle size between about 2 and 10 microns.

In accordance with another embodiment of the method of the present invention, the active pharmaceutical agent includes a first portion of the active pharmaceutical agent having an average particle size between about 1 and 4 microns, and a second portion of the active pharmaceutical agent having an average particle size between about 2 and 100 microns and preferably between about 10 and 100 microns, once again, preferably with the second portion of the active pharmaceutical agent having a particle size greater than that of the first active pharmaceutical agent.

In accordance with another embodiment of the method of the present invention, the drug-eluting product comprises a drug-eluting stent.

In accordance with another embodiment of the method of the present invention, the drug-eluting product comprises a degradable component, such as a degradable substrate, and a polymer coating disposed on the degradable component.

In accordance with one embodiment of this aspect of the present invention, the at least partially bioabsorbable element comprises a bioabsorbable polymer coating disposed on the degradable component. In another embodiment, the at least partially bioabsorbable element comprises a bioabsorbable polymer coating disposed on the degradable component, which preferably comprises a bioabsorbable substrate. In a preferred embodiment, the bioabsorbable substrate also comprises bioabsorbable polymers.

### BRIEF DESCRIPTION OF THE DRAWINGS

Figure 1 is a graphical representation of the kinetics of the degradation of the bioabsorbable polymer and the elution of drug samples of differing sizes therefrom.
Figure 2 is a graphical representation of the inflammation realized of a bioabsorbable implant containing both large and small particle size drug and only small particle size drug.

### DETAILED DESCRIPTION

The present invention is explained in greater detail below. This description is not intended to be a detailed catalog of all the different ways in which the invention may be implemented, or all the features that may be added to the instant invention. For example, features illustrated with respect to one embodiment may be incorporated into other embodiments, and features illustrated with respect to a particular embodiment may be deleted from that embodiment. In addition, numerous variations and additions to the various embodiments suggested herein will be apparent to those skilled in the art in light of the instant disclosure, which do not depart from the instant invention. Hence, the following specification is intended to illustrate some particular embodiments of the invention, and not to exhaustively specify all permutations, combinations and variations thereof.

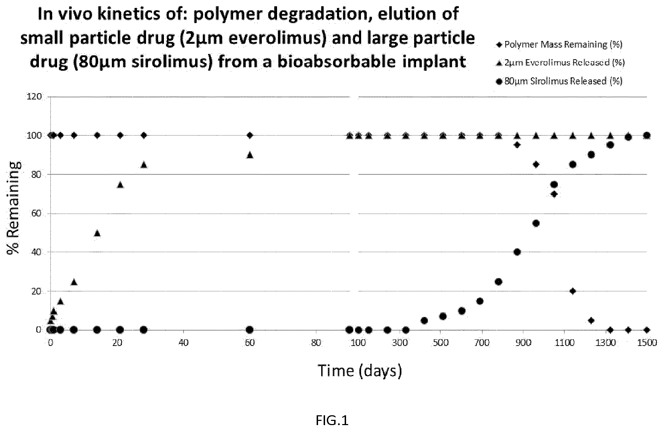

In its most general sense, the present invention provides a platform for the delivery of certain drugs to various locations in the body for a number of different purposes. In each of these embodiments of the present invention, the nature of the drug itself, and its particle size, is of critical importance. A drug is generally employed which is poorly soluble or insoluble in the aqueous solutions which it will encounter in the body, and which has a morphology which is preferably crystalline or semi-crystalline in nature, but which has a predetermined particle size distribution which is intended to obtain maximum effect on the target ailment, whether it be inflammation, cellular proliferation, or some other treatable condition. In one aspect, the present invention provides a drug formulation in which the drug will be substantially continuously released as long as the platform or degradable component on which it has been delivered remains. In another embodiment, the platform itself, when used, for example, as a stent or other implantable device, will degrade and cause inflammation or other difficulties, that is inflammation, or more specifically, cellular proliferation induced by the degradation of bioabsorbable materials, will result. Thus, in this embodiment, the active pharmaceutical ingredient or drug is formulated to have a morphology, a solubility and a particle size distribution so that it will be present and continuously or discontinuously released from the drug-eluting product, so long as the platform itself has not completely degraded. Thus, as an example, Fig. 1 shows a graphical representation of drug delivery from different particle sizes of the same drug (everolimus) over the timer period of the loss of mass from the bioabsorbable material as it degrades.

It is understood that other terms have been utilized in the past to describe these bioabsorbable materials and their degradation products. These include bioresorbable, bioabsorbable, biodegradable, and bioerodable, and are all meant to be included with the scope of bioabsorbable materials and their degradation products.

Thus, the platform itself can provide the vehicle for delivery of the active pharmaceutical ingredient, thus ranging from degradable carriers and conventional drug excipients to bioabsorbable polymers, bioabsorbable polymer coatings, and bioabsorbable metal components. In another aspect, however, the platform can comprise a stent or the like, coated with the active pharmaceutical ingredient. The stent can thus include a bioabsorbable polymer layer, or the stent itself can be prepared from a bioabsorbable polymer, so that upon degradation of the bioabsorbable polymer coating and/or the bioabsorbable polymer stent itself, the active pharmaceutical ingredient will still be present to treat inflammation or proliferation arising from the degradation of these portions of the platform.

In one aspect of the present invention an improved drug-eluting product is provided, such as a drug-eluting stent which not only includes bioabsorbable elements, but which is also configured so that the time course of drug delivery is explicitly controlled so as to provide a therapeutic level of drug at the same time that the bioabsorbable elements of the device are degrading. This control is basically affected by specifying the morphology, solubility and particle size of the drug which is being utilized.

In another aspect of the present invention, irrespective of the nature of the platform for the active pharmaceutical agent, as well as the at least partially bioabsorbable component, the active pharmaceutical agent itself is configured to exhibit prolonged elution, such that it will be released over an extended period of time of at least about 3 months, preferably at least about 6 months, more preferably at least about 9 months, even more preferably at least about 1 year, and most preferably for a period of at least about 18 months. For example, in the case of a drug-eluting stent, the active pharmaceutical agent can be applied along with a biodegradable polymer coating onto a stent which itself is not biodegradable, and the active pharmaceutical agent will nevertheless be released over these extended time periods.

In a particularly preferred embodiment of the present invention, a stent having a bioabsorbable scaffold and also containing a bioabsorbable coating on the scaffold is employed. Two of the major problem areas for the creation of inflammation/proliferation relate to the primary inflammation/proliferation which is associated with application of the stents themselves and late inflammation/proliferation which is associated with scaffold resorption in the case of bioabsorbable scaffolds. In accordance with the present invention, it is now possible to deal with both of these potential problems. In particular, this is accomplished by employing a bioabsorbable coating on the stent which has a crystalline or semi-crystalline morphology, a desired degree of insolubility, and which contains a range of drug particle sizes, specifically chosen to deal with both of these issues. In other words, by taking into consideration both the inflammation and/or proliferation which will occur early on, during, and after a stent implantation itself, and calculating the expected period of late proliferation by an understanding of the bioabsorbable scaffold itself, will thus have an impact on the period of time in which it is expected to be resorbed or eventually dissolved. Thus, various parameters can be selected to increase or decrease the time of resorption. With a polymeric scaffold, factors including the thickness of the stent elements, the composition of the polymers employed, the crystallinity of the polymers employed, etc., and in the case of metallic stents, the nature of the metal, the thickness of the metal, etc., can all have a bearing on the expected life of the stent. With this in mind, in accordance with the present invention, the drug or drugs employed in the bioabsorbable coating placed upon the scaffold can be specifically selected so that it is not only present at the outset, but it is also present throughout the period of resorption, *i.e.*, at least up until the entire scaffold has been resorbed, to deal with questions of inflammation and/or proliferation over that entire period. This is most principally accomplished by selection of particle size ranges for the particular drug involved. Preferably, two or more different portions of the drug or drugs used each having a different particle size range, can be employed. In this manner, the particular drug formulation utilized in the bioabsorbable coating on the stent can be specifically selected for the particular stent involved. In another embodiment, however, the drug or drugs can be employed in connection with the polymeric or metallic scaffold itself, and once again can be specifically selected so that it is present for a predetermined period of time, and in the case where a drug or drugs are also included in the bioabsorbable coating on the scaffold, the drug or drugs used in connection with the polymeric or metallic scaffold can be the same or different than the drug or drugs in the bioabsorbable coating. For example, a drug with a shorter period of dissolution can be incorporated in the bioabsorbable polymer coating, and a different drug, with a longer period of dissolution, such as at least 60 days, or at least a month, or at least 9 or 12 months, can be incorporated in the polymeric or metallic scaffold itself.

The drug-eluting elements of the present invention can include drug-eluting stents, but can also include other types of medical implants which are intended for insertion into the body of a human or animal subject. Thus, in addition to stents, including vascular stents, these can include electrodes, catheters, leads, implantable pacemakers, cardioverter or defibrillator housings, joints, screws, rods, ophthalmic implants, femoral pins, bone plates, graphs, and anastomotic devices, perivascular wraps, sutures, staples, shunts for hydrocephalus, dialysis grafts, colostomy bag attachment devices, ear drainage tubes, leads for pace makers and implantable cardioverters and defibrillators, vertebral disks, bone pins, suture anchors, hemostatic barriers, clamps, screws, plates, clips, vascular implants, tissue adhesives and sealants, tissue scaffolds, various types of dressings (e.g., wound dressings), bone substitutes, intraluminal devices, vascular supports, stent graphs, and membrane-based implants, such as hernia patches. The basic structure of the medical implants of this invention, including the degradable components or substrates, such as the stent structure itself, can be made from a durable polymer or a bioabsorbable polymer or other bioabsorbable material, such as a bioabsorbable metal component. The durable polymers from which these substrates can be produced can include various polymers or combinations of polymers. Examples of polymers that may be used in the present invention include, but are not limited to polycarboxylic acids, cellulosic polymers, proteins, polypeptides, polyvinylpyrrolidone, maleic anhydride polymers, polyamides, polyvinyl alcohols, polyethylene oxides, glycosaminoglycans, polysaccharides, polyesters, bacterial polyesters (PHB, PHV), polyurethanes, polystyrenes, copolymers, silicones, polyorthoesters, polyanhydrides, copolymers of vinyl monomers, polycarbonates, polyethylenes, polypropylenes, polylactic acids, polyglycolic acids, polycaprolactones, polyhydroxybutyrate valerates, polyacrylarides, polyethers, polyurethane dispersions, polyacrylates, acrylic latex dispersions, polyacrylic acid, mixtures and copolymers thereof. The polymers of the present invention may be natural or synthetic in origin, including gelatin, chitosan, dextrin, cyclodextrin, Poly(urethanes), Poly(siloxanes) or silicones, Poly(acrylates) such as poly(methyl methacrylate), poly(butyl methacrylate), and Poly(2-hydroxy ethyl methacrylate), Poly(vinyl alcohol) Poly(olefins) such as poly(ethylene), poly(isoprene), halogenated polymers such as Poly(tetrafluoroethylene)--and derivatives and copolymers such as those commonly sold as Teflon.RTM. products, Poly(vinylidine fluoride), Poly(vinyl acetate), Poly(vinyl pyrrolidone), Poly(acrylic acid), Polyacrylamide, Poly(ethylene-co-vinyl acetate), Poly(ethylene glycol), Poly(propylene glycol), Poly(methacrylic acid), Poly(dimethyl)-siloxane, Polyethyene terephthalate, Polyethylene-vinyl acetate copolymer (PEVA), Ethylene vinyl alcohol (EVAL), Ethylene vinyl acetate (EVA), Poly(styrene-b-isobutylene-b-styrene) (SIBBS), Phosophorycholine (PC), styrene-isobutylene, fluorinated polymers, polyxylenes (PARYLENE), tyrosine based polycarbonates, tyrosine based polyarylates, poly(trimethylene carbonate), hexafluoropropylene, vinylidene fluoride, butyl methacrylate, hexyl methacrylate, vinyl pyrrolidinone, vinyl acetate, etc.

The bioabsorbable and/or resorbable polymers which can be used for these substrates, such as the stents of this invention, including the following, combinations, copolymers and derivatives of the following: Polylactides (PLA), Poly(L-lactides) (PLLA), Poly(L-lactide-co-D,L-lactide) (PLDLA), Poly(D-lactide) (PDLA), Poly(D,L-lactide) (PDLLA), Polyglycolides (PGA), Poly(lactide-co-glycolides) (PLGA) and Poly(L-lactide-co-glycolide) (PLLGA). Polyanhydrides, Polyorthoesters, polyhydroxyalkanoates, such as poly(hydroxybutyrate)(PHB), poly(hydroxyvalerate) (PHV), and poly(hydroxybutyrate-co-valerate) (PHOV), polylactones, such as polycaprolactone (PCL), poly(lactide-co-caprolactone), polyphosphoesters, polyacrylates, Poly(N-(2-hydroxypropyl)methacrylamide), Poly(1-aspartamide), Polyethyleneoxide/polybutylene terephthalate copolymer, poly(dioxanone) (PDO), poly(glycolide-co-trinethylene carbonate (PGA-TMC)), polyanhydrides, poly(propylene fumarate), and polydioxanone.

Irrespective of the nature of the substrate utilized, in addition, various metals can be considered to be biostable or bioerodable for use in connection with these stents. These metals are considered to be bioerodable because they tend to erode or corrode relatively rapidly when implanted or when exposed to bodily fluids. Representative examples of biodegradable metals that may be used to fabricate an implantable medical device may include, but are not limited to, magnesium, zinc, and iron. In addition, other such metals can include titanium, zirconium, niobium, tantalum, silicon, and alloys thereof, such as with lithium, sodium, potassium, and calcium. As an example, the time frame for erosion for an erodible metallic stent can result in complete erosion between about a week and about three months, or more narrowly between about one month and about two months. In addition, the erodible metal or metal alloys can be selected for longer residual times, such as one year or longer, such as Magnesium alloys in particular. It is also noted that, in addition to the inflammation/proliferation created by certain of these substrates, with these metals there is also the potential calcification which can occur after degradation, and the use of an active pharmaceutical agent which can combat such calcification should also be considered in connection with this invention.

Application of a polymer coating to these degradable components or substrates, such as these stent structures, have conventionally been carried out by processes such as dipping, spraying, vapor deposition, plasma polymerization, and electrodeposition. These processes have had several difficulties, including the use of solvents and the like. In light of this, processes and products have been developed, such as those shown in U.S. Patent No. 8,298,565 by the Assignee of the present application, in which a specific process for coating these products with an active pharmaceutical ingredient, such as rapamycin, in combination with a polymer coating, and in which the rapamycin is in the crystalline or semi-crystalline form, have been developed. These processes generally include depositing a pharmaceutical agent in dry powder form onto the substrate, depositing at least one polymer in dry powder form onto the substrate, and sintering the coating under conditions such that the morphology of the solid pharmaceutical polymers particles are not substantially modified. In this regard, the entire disclosure of U.S. Patent No. 8,298,565 is incorporated herein by reference.

The present invention can also be applied to one embodiment in which a durable metallic stent or substrate is employed, and is then coated with a polymer composition. We have specifically discussed above the use of a bioabsorbable polymer coating in connection with the active pharmaceutical agents of this invention. Another embodiment would include a durable metallic or polymeric stent which is coated with a durable or permanent polymer composition. Thus, this particular product does not include a biodegradable component, but nevertheless, the use of this product in connection with the active pharmaceutical agents of the present invention can also have an important utility. That is, the particle size of the active pharmaceutical agent can still be chosen to extend the time for drug delivery into the system. This can aid in the long-term maintenance of the open nature of the stented artery itself. The particular drugs which are considered to be useful in connection with this embodiment of the present invention can include anti-inflammatory agents, such as dexamethasone, prednisolone, corticosterone, budesonide, estrogen, sulfasalazine, mesalomine, and analogs thereof; anti-neoplastic/anti-proliferative/anti-miotic agents, such as paclotaxcil, 5-fluor urosil; cysplatin, vinblastine, vincristine, epothilones, endostatin, angiostatin, thymidine kinase inhibitors, and analogs thereof, and preferably macrolide immunosuppressive (limus) drugs, such as rapamycin, everolimus, rapamycin analogs, tacrolimus, temsirolimus, zotorolimus, or derivatives, isomers, racemates, diesterioisomers, prodrugs, hydrates, esters, or other analogs thereof, and in particular, using these drugs with a particle size approaching 100 microns (or the approximate size of the struts on stents or substrates hereof), can be particularly useful in this embodiment.

In yet another embodiment to the present invention, in connection with durable metallic and/or polymeric stents, the substrates or stents themselves can include reservoirs, grooves, pores, nano-scale structures, and the like. Thus, the active pharmaceutical agents can be incorporated into the substrates by being placed within these reservoirs, grooves, pores, and nano-scale structures for drug delivery purposes. Once again, the active pharmaceutical agents can be incorporated into these reservoirs or grooves by using a polymer, preferably a bioabsorbable polymer, in order to maintain the active pharmaceutical agents therein and to further control their elution profiles.

Another application of the present invention relates to peripheral arteries and blood vessels. Thus, self-expanding stents can be employed in the periphery, and can incorporate into them the elements of the present invention, including the bioabsorbable component and an active pharmaceutical ingredient. Yet another application of the present invention relates to stent grafts and the like. These are stents, most-commonly self-expanding nitinol stents, which are covered with a fabric material that is knitted from a biostable polymer such as expanded polytetrafluoroethylene (ePTFE), polyethylene terephthalate (Dacron®) and polyurethane. These can then be coated with a polymer coating containing drug particles.

As is discussed above, the drug-eluting elements of the present invention can include membrane-based implants, such as hernia patches and the like. In these embodiments, once again the use of an active pharmaceutical agent along with a bioabsorbable polymer material can take significant advantage of this invention. In these environments, if a bioabsorbable polymer is used to fix the active pharmaceutical agent to the surface of the implant or patch, then the particle size of the active pharmaceutical agent can be selected to counteract potential inflammation or infection caused by the degradation of the bioabsorbable polymer over extended time periods.

The active pharmaceutical agents which can be employed in connection with this invention include any of a variety of drugs or pharmaceutical compounds that can be used as active agents to prevent or treat a disease (meaning any treatment of a disease in a mammal, including preventing the disease, i.e. causing the clinical symptoms of the disease not to develop; inhibiting the disease, i.e. arresting the development of clinical symptoms; and/or relieving the disease, i.e. causing the regression of clinical symptoms). It is possible that the pharmaceutical agents of the invention may also comprise two or more drugs or pharmaceutical compounds. Pharmaceutical agents, include but are not limited to antirestenotic agents, antidiabetics, analgesics, antiinflammatory agents, antirheumatics, antihypotensive agents, antihypertensive agents, psychoactive drugs, tranquilizers, antiemetics, muscle relaxants, glucocorticoids, agents for treating ulcerative colitis or Crohn's disease, antiallergics, antibiotics, antiepileptics, anticoagulants, antimycotics, antitussives, arteriosclerosis remedies, diuretics, proteins, peptides, enzymes, enzyme inhibitors, gout remedies, hormones and inhibitors thereof, cardiac glycosides, immunotherapeutic agents and cytokines, laxatives, lipid-lowering agents, migraine remedies, mineral products, otologicals, anti parkinson agents, thyroid therapeutic agents, spasmolytics, platelet aggregation inhibitors, vitamins, cytostatics and metastasis inhibitors, phytopharmaceuticals, chemotherapeutic agents and amino acids. Examples of suitable active ingredients are acarbose, antigens, beta-receptor blockers, non-steroidal antiinflammatory drugs {NSAIDS}, cardiac glycosides, acetylsalicylic acid, virustatics, aclarubicin, acyclovir, cisplatin, actinomycin, alpha- and beta-sympatomimetics, (dmeprazole, allopurinol, alprostadil, prostaglandins, amantadine, ambroxol, amlodipine, methotrexate, S-aminosalicylic acid, amitriptyline, amoxicillin, anastrozole, atenolol, azathioprine, balsalazide, beclomethasone, betahistine, bezafibrate, bicalutamide, diazepam and diazepam derivatives, budesonide, bufexamac, buprenorphine methadone, calcium salts, potassium salts, magnesium salts, candesartan, carbamazepine, captopril, cefalosporins, cetirizine, chenodeoxycholic acid, ursodeoxycholic acid, theophylline and theophylline derivatives, trypsins, cimetidine, clarithromycin, clavulanic acid, clindamycin, clobutinol, clonidine, cotrimoxazole, codeine, caffeine, vitamin D and derivatives of vitamin D, colestyramine, cromoglicic acid, coumarin and coumarin derivatives, cysteine, cytarabine, cyclophosphamide, ciclosporin, cyproterone, cytabarine, dapiprazole, desogestrel, desonide, dihydralazine, diltiazem, ergot alkaloids, dimenhydrinate, dimethyl sulphoxide, dimeticone, domperidone and domperidan derivatives, dopamine, doxazosin, doxorubizin, doxylamine, dapiprazole, benzodiazepines, diclofenac, glycoside antibiotics, desipramine, econazole, ACE inhibitors, enalapril, ephedrine, epinephrine, epoetin and epoetin derivatives, morphinans, calcium antagonists, irinotecan, modafinil, orlistat, peptide antibiotics, phenyloin, riluzoles, risedronate, sildenafil, topiramate, macrolide antibiotics, oestrogen and oestrogen derivatives, progestogen and progestogen derivatives, testosterone and testosterone derivatives, androgen and androgen derivatives, ethenzamide, etofenamate, etofibrate, fenofibrate, etofylline, etoposide, famciclovir, famotidine, felodipine, fenofibrate, fentanyl, fenticonazole, gyrase inhibitors, fluconazole, fludarabine, fluarizine, fluorouracil, fluoxetine, flurbiprofen, ibuprofen, flutamide, fluvastatin, follitropin, formoterol, fosfomicin, furosemide, fusidic acid, gallopamil, ganciclovir, gemfibrozil, gentamicin, ginkgo, Saint John's wort, glibenclamide, urea derivatives as oral antidiabetics, glucagon, glucosamine and glucosamine derivatives, glutathione, glycerol and glycerol derivatives, hypothalamus hormones, goserelin, gyrase inhibitors, guanethidine, halofantrine, haloperidol, heparin and heparin derivatives, hyaluronic acid, hydralazine, hydrochlorothiazide and hydrochlorothiazide derivatives, salicylates, hydroxyzine, idarubicin, ifosfamide, imipramine, indometacin, indoramine, insulin, interferons, iodine and iodine derivatives, isoconazole, isoprenaline, glucitol and glucitol derivatives, itraconazole, ketoconazole, ketoprofen, ketotifen, lacidipine, lansoprazole, levodopa, levomnethadone, thyroid hormones, lipoic acid and lipoic acid derivatives, lisinopril, lisuride, lofepramine, lomustine, loperamide, loratadine, maprotiline, mebendazole, mebeverine, meclozine, mefenamic acid, mefloquine, meloxicam, mepindolol, meprobamate, meropenem, mesalazine, mesuximide, metamizole, metformin, methotrexate, methylphenidate, methylprednisolone, metixene, metoclopramide, metoprolol, metronidazole, mianserin, miconazole, minocycline, minoxidil, misoprostol, mitomycin, mizolastine, moexipril, morphine and morphine derivatives, evening primrose, nalbuphine, naloxone, tilidine, naproxen, narcotine, natamycin, neostigmine, nicergoline, nicethamide, nifedipine, niflumic acid, nimodipine, nimorazole, nimustine, nisoldipine, adrenaline and adrenaline derivatives, norfloxacin, novamine sulfone, noscapine, nystatin, ofloxacin, olanzapine, olsalazine, omeprazole, omoconazole, ondansetron, oxaceprol, oxacillin, oxiconazole, oxymetazoline, pantoprazole, paracetamol, paroxetine, penciclovir, oral penicillins, pentazocine, pentifylline, pentoxifylline, perphenazine, pethidine, plant extracts, phenazone, pheniramine, barbituric acid derivatives, phenylbutazone, phenytoin, pimozide, pindolol, piperazine, piracetam, pirenzepine, piribedil, piroxicam, pramipexole, pravastatin, prazosin, procaine, promazine, propiverine, propranolol, propyphenazone, prostaglandins, protionamide, proxyphylline, quetiapine, quinapril, quinaprilat, ramipril, ranitidine, reproterol, reserpine, ribavirin, rifampicin, risperidone, ritonavir, ropinirole, roxatidine, roxithromycin, ruscogenin, rutoside and rutoside derivatives, sabadilla, salbutamol, salmeterol, scopolamine, selegiline, sertaconazole, sertindole, sertralion, silicates, sildenafil, simvastatin, sitosterol, sotalol, spaglumic acid, sparfloxacin, spectinomycin, spiramycin, spirapril, spironolactone, stavudine, streptomycin, sucralfate, sufentanil, sulbactam, sulphonamides, sulfasalazine, sulpiride, sultamicillin, sultiam, sumatriptan, suxamethonium chloride, tacrine, tacrolimus, taliolol, tamoxifen, taurolidine, tazarotene, temazepam, teniposide, tenoxicam, terazosin, terbinafine, terbutaline, terfenadine, terlipressin, tertatolol, tetracyclins, teryzoline, theobromine, theophylline, butizine, thiamazole, phenothiazines, thiotepa, tiagabine, tiapride, propionic acid derivatives, ticlopidine, timolol, tinidazole, tioconazole, tioguanine, tioxolone, tiropramide, tizanidine, tolazoline, tolbutamide, tolcapone, tolnaftate, tolperisone, topotecan, torasemide, antioestrogens, tramadol, tramazoline, trandolapril, tranylcypromine, trapidil, trazodone, triamcinolone and triamcinolone derivatives, triamterene, trifluperidol, trifluridine, trimethoprim, trimipramine, tripelennamine, triprolidine, trifosfamide, tromantadine, trometamol, tropalpin, troxerutine, tulobuterol, tyramine, tyrothricin, urapidil, ursodeoxycholic acid, chenodeoxycholic acid, valaciclovir, valproic acid, vancomycin, vecuronium chloride, Viagra, venlafaxine, verapamil, vidarabine, vigabatrin, viloazine, vinblastine, vincamine, vincristiine, vindesine, vinorelbine, vinpocetine, viquidil, warfarin, xantinol nicotinate, xipamide, zafirlukast, zalcitabine, zidovudine, zolmitriptan, zolpidem, zoplicone, zotipine and the like. See, e.g., U.S. Pat. No. 6,897,205; see also U.S. Pat. Nos. 6,838,528; 6,497,729.

Examples of therapeutic agents employed in conjunction with the invention include, rapamycin, 40-O-(2-Hydroxyethyl)rapamycin (everolimus), 40-O-Benzyl-rapamycin, 40-O-(4'-Hydroxymethyl)benzyl-rapamycin, 40-O-[4'-(1,2-Dihydroxyethyl)]benzyl-rapamycin, 40-O-Allyl-rapamycin, 40-O-[3'-(2,2-Dimethyl-1,3-dioxolan-4(S)-yl)-prop-2'-en-1'-yl]-rapamycin, (2':E,4'S)-40-O-(4',5'-Dihydroxypent-2'-en-1'-yl)-rapamycin, 40-O-(2-Hydroxy)ethoxycarbonylmethyl-rapamycin, 40-O-(3-Hydroxy)propyl-rapamycin, 40-O-(6-Hydroxy)hexyl-rapamycin, 40-O-[2-(2-Hydroxy)ethoxy]ethyl-rapamycin, 40-O-[(3S)-2,2-Dimethyldioxolan-3-yl]methyl-rapamycin, 40-O-[(2S)-2,3-Dihydroxyprop-1-yl]-rapamycin, 40-O-(2-Acetoxy)ethyl-rapamycin, 40-O-(2-Nicotinoyloxy)ethyl-rapamycin, 40-O-[2-(N-Morpholino)acetoxy]ethyl-rapamycin, 40--O-(2-N-Imidazolylacetoxy)ethyl-rapamycin, 40-O-[2-(N-Methyl-N'-piperazinyl)acetoxy]ethyl-rapamycin, 39-O-Desmethyl-39,40-O,O-ethylene-rapamycin, (26R)-26-Dihydro-40-O-(2-hydroxy)ethyl-rapamycin, 28-O-Methyl-rapamycin, 40-O-(2-Aminoethyl)-rapamycin, 40-O-(2-Acetaminoethyl)-rapamycin, 40-O-(2-Nicotinamidoethyl)-rapamycin, 40-O- (2-(N-ethyl-imidazo-2'-ylcarbethoxamido)ethyl)-rapamycin, 40-O-(2-Ethoxycarbonylaminoethyl)-rapamycin, 40-O-(2-Tolylsulfonamidoethyl)-rapamycin, 40-O-[2-(4',5'-Dicarboethoxy-1',2',3'-triazol-1'-yl)-ethyl]-rapamycin, 42-Epi-(tetrazolyl)rapamycin (tacrolimus), and 42-[3-hydroxy-2'-(hydroxymethyl)-2-methylpropanoate]rapamycin (temsiro limus).

These limus drugs are particularly suitable for the present invention because of their poor solubility and availability in a crystalline morphology; both factors that allow dissolution of the drug to be a rate limiting factor in the time course of drug delivery. This dissolution-controlled drug delivery then is further controlled by the selection of specific particle sizes and/or particle size distribution(s) in accordance with the present invention. Other such drugs which can thus be used in accordance with this invention include, for example, corticosteroids, such as common natural hormones, such as corticosterone, cortisone, and aldosterone. These also include first generation corticosteroids, such as cortisol, prednisone, and dexamethasone, as well as second degeneration corticosteroids, such as triamcinolone acetonide, fluticasone proprionate, beclomethasone. In addition, certain non-steroidal anti-inflammatory drugs, such as the cox-2 inhibitors which are also quite insoluble, could be used in connection with the present invention, including celecoxib and rofecoxib, as well as etoricoxib. Indeed, the local delivery of these drugs can be highly beneficial, not only by decreasing side effects and increasing potency, but also potentially ameliorating the systemic toxicity of some of these drugs. In addition, the PPARγ and PPARα (peroxisome proliferation activated receptor) agonists, such as pioglitazone are useful in accordance with this invention. Alpha agonists are primarily fibrate drugs, such as clofibrate, gemfibrozil, suprofibrate, bizafibrate, and fenofibrate. The PPARγ agonists include thiazolidinediones.

The active ingredients may, if desired, also be used in the form of their pharmaceutically acceptable salts or derivatives (meaning salts which retain the biological effectiveness and properties of the compounds of this invention and which are not biologically or otherwise undesirable), and in the case of chiral active ingredients it is possible to employ both optically active isomers and racemates or mixtures of diastereoisomers.

In another embodiment of the present invention, multiple drugs and drug combinations can be employed within the context of this invention. For example, one drug can be employed which has an initial elution profile for a relatively short period of time, and a second or multiple other drugs can be employed having a selected particle size in accordance with this invention such that it has an elution profile such that the drug persists for an extended period of time, such as up to or beyond the point where the degradation products from the bioabsorbable material used in the particular product in question have been exhausted. As an example, the initial drug, which can include small particles, can be a limus-type drug, such as sirolimus, which provides an anti-restonetic affect during the early stages of implantation, such as for example for up to 4 to 12 weeks thereafter. The additional drug or drugs having a larger particle size can comprise an anti-inflammatory drug, such as those discussed above, to provide a local therapeutic affect much later in the process, and most particularly while the bioabsorbable material is in the process of degrading, again so as to counteract the pro-inflammatory tendency of these degradation products, and to thus avoid the potential negative response elicited by these degradation products in the local tissues. There are, of course, a large number of specific combinations of drugs and drug particle sizes which can accomplish these results. Indeed, these can include the same drug with two different particle sizes, or a second drug, such as a different macrolide antibiotic which has a longer tissue half-life than the drug used in the initial elution. The drug or drugs which are used in the latter part of the elution profile can have longer drug release profiles based upon large or larger particle sizes, but also upon a combination of selected particle sizes with other drugs, such as anti-inflammatory steroids, non-steroidal anti-inflammatories, and the like. In general, if the second or later drugs have crystal structures, they may be selected to have sufficient stability to thus allow long residence with the micronized particles hereof. Thus, while a factor, the larger size of the drug used for the latter part of the elution profile may not necessarily be the case vis-à-vis the particle size for the first drug used for the initial phase of the elution profile. A combination of all of these factors will thus lead to a product in which the drug elution profile accomplishes all of the results discussed herein, including protection against inflammation and proliferation for as long as necessary with the particular stent at issue.

While the use of crystalline drugs, such as crystalline rapamycin, are an important embodiment of the present invention, the present invention is applicable to a much wider variety of drug morphologies. That is, the drugs themselves can be in a crystalline, amorphous, or semi-crystalline form, the critical element in controlling the time course of drug delivery being the particle size, and the dissolution kinetics of these drug components. In that regard, dissociation from a given particle can be rate-limiting for drug availability regardless of whether the drug is crystalline or amorphous. The dissociation rates may, and in all likelihood will, be different between the two, but the underlying concept that small particles release faster for a shorter time than larger particles, remains the same. However, with amorphous drugs also having relatively low solubility, there is a far lower extent of control, that is the case with crystalline or semi-crystalline drugs. Furthermore, with respect to the semi-crystalline drugs, the relationship to particle size will be dependent upon the kinetics and thermodynamics of solubility of the drug in a given morphology. In general, the larger the particle size the greater prolonging of drug delivery which will be realized, particularly as compared to smaller particle sizes with drugs of similar dissolution properties. Thus, in producing the product of this invention, based upon the particular nature of the bioabsorbable elements used in that product, be they bioabsorbable polymer coatings and/or bioabsorbable substrates themselves, one can then determine the particular total reabsorption time for these elements, thus calculating the time when the degradation products of these bioabsorbable elements remain in the bloodstream, and then a particular particle size or particle size distribution can be selected so as to maintain these active pharmaceutical agents in the bloodstream for at least that time period. In general, the ranges of particle sizes which can be employed can vary. For example, from 20 nanometers up to about 100 microns. Within that range, particular particle sizes for particular products can range, for example, from 20 to 100 nanometers; from 100 nanometers to 1 micron, from 1 to 10 microns, from 10 to 50 microns, from 50 to 100 microns, a figure which approaches the dimension of stent struts themselves.

In a similar vein, the poor solubility of the active pharmaceutical agents of this invention is yet another factor which can be taken into account when preparing a particular product for a particular indication. Thus, the solubility of the drug will directly impact upon their dissolution time. The more poorly soluble the drugs are, the longer such dissolution will take, and as is the case with respect to the crystalline nature of these drugs as discussed above, the same will be true with respect to the relative insolubility of the particular drug in question.

Another class of drugs which can be employed in connection with this invention are so-called pro-healing drugs or active agents which will induce or aid in the formation of either smooth muscle cells or endothelium. This would generally comprise proteins, peptides, or enzymes.

Another specific drug which can be useful in connection with the present invention is the drug paclitaxel. Paclitaxel particles, whether crystalline or amorphous, will dissociate extremely slowly. Indeed, the dissociation may be so slow that it may be necessary to use composite particles with a component that will dissolve faster in order to facilitate release of the paclitaxel. In any event, the use of paclitaxel, like the other drugs discussed above, can find particular use by selecting particle size distributions in the manner discussed herein.

With these parameters, it is also possible to select narrower particle size distributions for specific drug elution profiles. That is, narrower particle size distributions can provide for narrower "windows" of drug release or delivery. This can apply to the use of narrow particle distributions for both the particles responsible for the initial portion of the drug elution profile and the particles responsible for later portion(s) of the drug elution profile to provide for these narrow windows at both the period or early release and the period of late release. These time periods for release can relate to the use of smaller or larger particle size distributions, as where similar drugs are utilized, but that is not necessarily the case as discussed above in connection with other relevant factors, such as the crystallinity or other morphology of the drug on the relative insolubility of the drug. In effect, this is a method of providing a spike or spikes of drug delivery over the course of time. It can also be applied to particular short duration products, such as a drug-coated balloon, for example, where one requires delivery of a drug and/or sustained-release drug formulations into the arterial tissue in a very short period of time.

In general, however, the broader particle size distributions appear to have greater potential application, and can create a more blended elution rate. Using these broad particle sized distributions, a smoother or continuous drug delivery profile can be attained over an extended period of time. Particular applications include those discussed above, such as with self-expanding peripheral stents or bioabsorbable scaffolds, which require continuous long duration drug availability and/or both early and late segment drug application, such as in the case of a biodegradable scaffold, again as discussed above.

Furthermore, blends of different particle sizes can be included in a single product. Thus, smaller particles can be included to provide drug in the early time course, while larger particle sizes can provide drug at later times. In preferred embodiments where at least two particle size ranges are employed, a first or smaller particle size range of from about 100 nm to 10 microns, preferably 1 to 4 µ can be used along with a larger particle size range, preferably from 1 to 100 µ, and preferably from 2 to 10 µ.

The present invention may be more fully appreciated with reference to the following illustrative examples as follows:

### Illustrative Example #1

According to the present invention a drug delivery implant is formed to allow for two distinct periods of local release of a drug such as sirolimus. Sirolimus, also known as rapamycin, was obtained as a bulk powder from LC Laboratories. The sirolimus is micronized by jet milling to a mean particle size of 80µm (to be referred to as the Large Particle Drug). Everolimus is obtained as a bulk powder from LC Laboratories and is micronized by jet milling to a mean particle size of 2µm (to be referred to as the Small Particle Drug). 85:15 mole ratio PLGA is obtained from Lactel Absorbable Polymers (Standard Product Number: B6006-1). The PLGA and the Large Particle Drug and the Small Particle Drug are mixed in a 5:1:1 ratio from which a 2mm diameter rod is formed by extrusion. Drug delivery implants are prepared by cutting 10mm length sections of this rod and sterilization by ethylene oxide exposure.

The resulting rods are surgically implanted intramuscularly in domestic swine. The in vivo pharmacokinetics of drug delivery is monitored at various time points by harvesting of the implant and surrounding ∼10mm of tissues, separation of any remnants of the implant and analysis of the tissues by chromatographic methods to assess the quantity of drug. The amount of drug quantified in tissue is then reported as a % of the amount loaded in the original implant, normalized to 100% after three analyses in a row report the same (maximum) value +/- 10%. The amount of polymer is assessed by extraction of the tissue sample and any remaining portion of the implant with chloroform, and then quantified gravimetrically. Results are reported as % remaining based on the mass of the original implant. Results are illustrated in the graph shown in Fig. 1.

### Illustrative Example #2

The materials (PLGA, Large Particle Drug and Small Particle Drug) of Example #1 are used to prepare samples to assess in vivo tissue response. 2mm x 10mm rods are prepared as follows:
1. Test Device: 5:1:1 ratio, same as example #1
2. Control Device A: Same composition with the exclusion of the Large Particle Drug (*e.g.* 5:1 ratio of PLGA to Small Particle Drug)

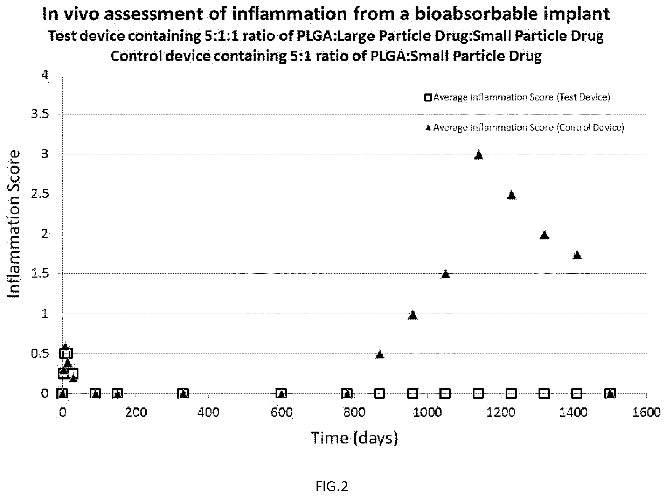

The tissue response to these implants is evaluated by the same animal model as Example #1 with histopathology assessment qualifying the extent of inflammation. Inflammation is scored on a scale of 0 → 4 with 0 being essentially absent inflammation and 4 being excessive inflammation observed in the histopathology samples. The results are presented in Fig. 2.

These results demonstrate the significance of the use of the small particle size, in this case in connection with a crystalline drug having a very low solubility in terms of the *in vivo* kinetics, and the continued elution of drug after polymer degradation. The results also demonstrate these results by means of the inflammation which is shown to be far superior in connection with the use of the small particle size drug, as compared to the combination of large and small particle size drug.

The above detailed description sets forth various features and functions of the disclosed drug-elution products and their method of manufacture. While various embodiments have been disclosed herein, other aspects and embodiments will be apparent to those skilled in the art. The various aspects and embodiments disclosed herein are for purposes of illustration and are not intended to be limiting, with the true scope and spirit being understood by the following claims.

## Claims

1. A drug-eluting product comprising a degradable component having a predetermined period of degradation and including an active pharmaceutical agent having a morphology, a solubility and an average particle size which are selected so that said active pharmaceutical agent continues to dissolve over the entire predetermined period of degradation of said degradable component, said morphology comprising a crystalline, semi-crystalline, or amorphous morphology, said solubility comprising a solubility of less than 100 µg/ml and said average particle size being greater than about 100 nm.

2. The drug-eluting product of claim 1, wherein said active pharmaceutical agent has an average particle size of between about 1 µm and 10 µm.

3. The drug-eluting product of claim 1 or 2, wherein said active pharmaceutical agent includes a first portion of said active pharmaceutical agent having a particle size of between about 1 µm and 4 µm, and a second portion of said active pharmaceutical agent having a particle size of between about 2 µm and 10 µm.

4. The drug-eluting product of claim 1, 2, or 3, including a polymer coating disposed on said degradable component.

5. The drug-eluting product of claim 1, 2, 3 or 4 wherein said drug-eluting product includes an at least partially bioabsorbable element, wherein said at least partially bioabsorbable element comprises a bioabsorbable polymer coating disposed on said degradable component.

6. The drug-eluting product of claim 5, wherein said at least partially bioabsorbable element comprises a bioabsorbable polymer disposed on said degradable component, and said degradable component comprises a bioabsorbable substrate.

7. The drug-eluting product of any of claims 1-6, wherein said degradable component comprises a bioabsorbable polymer, a bioabsorbable metal, a degradable carrier, an excipient, or a pill core.

8. The drug-eluting product of any of claims 1-7, wherein said active pharmaceutical agent has a solubility comprising a solubility of less than 100 µg/ml, and has a particle size distribution comprising a particle size greater than about 0.5 µm.

9. The drug-eluting product of any of claims 1-8 comprising a drug-eluting stent.

10. The drug-eluting product of any of claims 1-9, wherein said active pharmaceutical agent comprises a first pharmaceutical agent, and including a second pharmaceutical agent associated with said degradable component.

11. The drug-eluting product of any of claims 1-10, wherein said drug-eluting product comprises implants, oral pharmaceuticals, injectables, trans-mucosals, inhalants, or topicals.

12. A method of preparing a drug-eluting product comprising
- providing an at least partially bioabsorbable element; and
- including an active pharmaceutical agent in said at least partially bioabsorbable element, said active pharmaceutical agent having a morphology, a solubility and a predetermined configuration selected so that said active pharmaceutical agent will continue to dissolve during the entire biodegradation of said biodegradable element, said morphology comprising a crystalline, amorphous, or semi-crystalline morphology, said solubility comprising a solubility of less than 100 µg/ml, and said predetermined configuration comprising a soluble particle size large enough to permit said active pharmaceutical agent to continue to dissolve for a period of greater than about one year.

13. The method of claim 12, including selecting an active pharmaceutical agent having an average particle size large enough so that said active pharmaceutical agent continues to dissolve during the biodegradation of said biodegradable element.

14. The method of claim 12 or 13, wherein said active pharmaceutical agent has an average particle size greater than about 0.5 µm.

15. The method of claim 12, 13, or 14, wherein said active pharmaceutical agent includes a first portion of said active pharmaceutical agent having a particle size between about 0.5 µm and 10 µm, and a second portion of said active pharmaceutical agent having a particle size between about 0.5 µm and 100 µm.
